# EUROPÄISCHE PATENTSCHRIFT

(11) **EP 1 865 580 B1**
(45) Veröffentlichungstag und Bekanntmachung des Hinweises auf die Patenterteilung: **19.01.2011**
(21) Anmeldenummer: 07008007.2
(22) Anmeldetag: 19.04.2007
(51) Int. Cl.: H01R 13/52

(54) **Verbindungsvorrichtung und Verfahren zu deren Einsatz**
Connecting device and method for application
Dispositif de liaison et son procédé d'utilisation

(30) Priorität: 08.06.2006 DE 102006026720
(43) Veröffentlichungstag der Anmeldung: 12.12.2007
(73) Patentinhaber: Odu Steckverbindungssysteme GmbH & Co. KG, 84453 Mühldorf (DE)
(72) Erfinder: Weigand, Josef, 84431 Heldenstein (DE)
(74) Vertreter: Leinweber & Zimmermann

(56) Entgegenhaltungen:
- US-A- 5 662 488
- US-A1- 2005 177 199

## Beschreibung

Die Erfindung betrifft eine Verbindungsvorrichtung für medizinische Zwecke zur Übertragung optischer und/oder elektrischer Signale.

Derartige Verbindungsvorrichtungen sind im medizinischen Bereich gut bekannt. Sie werden dazu verwendet, Signale aus dem Körperinneren eines Patienten nach außen und/oder umgekehrt zu übertragen. Dies ist beispielsweise erforderlich, wenn dem Patienten ein medizinischer Apparat wie beispielsweise eine Insulinpumpe eingesetzt wird, und der Apparat z. B. zu Steuerungszwecken von außen ansprechbar sein soll und/oder Signale nach außen abgeben können soll, z. B. um den Status des Apparates anzugeben. Dazu führt das Kabel von dem eingepflanzten Apparat durch eine Körperöffnung nach außen, und die Kuppeleinrichtung wird an ein passendes Gegenstück angekoppelt, das wiederum mit einer Steuereinrichtung und/oder Auswerteeinrichtung verbunden werden kann.

Zur Vorbereitung dieses Einsatzes muß das Kabel der Verbindungsvorrichtung entsprechend verlegt werden. Dazu wird die Verbindungsvorrichtung durch eine erste Körperöffnung eingeführt und durch eine zweite Körperöffnung aus dem Körperinneren nach außen ausgeführt. Dabei können die Körperöffnungen zusammenfallen, es sind aber üblicherweise unterschiedliche Öffnungen. Dies hängt damit zusammen, daß für die Öffnung für den Ausgang der Verbindungsvorrichtung eine geeignete vorbestimmte Lage bevorzugt wird, unabhängig davon, in welchem Körperbereich der medizinische Apparat eingepflanzt ist.

Zum Legen der elektrischen und/oder optischen Verbindung ist also ein invasiver Eingriff nötig, im Zuge dessen eine Durchgangsöffnung in den Körper des Patienten eingebracht wird. US 5,662,488 offenbart ein Verbindungssystem. US 2005/0 177 199 A1 offenbart einen Adapter für ein Implantierbares Kabel.

Der Erfindung liegt die Aufgabe zugrunde, eine Verbindungsvorrichtung bereitzustellen, die eine bessere Minimierung des oben erläuterten Eingriffs ermöglicht, insbesondere eine Minimierung der Abmessungen der Durchgangsöffnung.

Diese Aufgabe wird gemäß der Erfindung durch eine Verbindungsvorrichtung der eingangs erläuterten Art gelöst, die die Merkmale des Auspruchs 1 aufweist.

Bei der erfindungsgemäßen Verbindungsvorrichtung können demnach Innenteil und Kabel schonend in den menschlichen Körper eingeführt bzw. aus ihm herausgeführt werden, da der Außenteil nicht durch die entsprechende(n) Öffnung(en) miteingeführt bzw. ausgeführt werden muß, sondern erst anschließend an dem Innenteil angebracht wird. So werden Körperbereiche des Patienten, gegen die der Außenteil bei Einsatz herkömmlicher Verbindungsvorrichtungen schlägt, drängt und/oder an denen er zieht, geringeren Belastungen ausgesetzt. Insbesondere ist es möglich, kleinere als herkömmlicherweise nötige Einschnitte zur Herstellung der Öffnung(en) zu machen, um den invasiven Eingriff so minimal wie möglich ausfallen zu lassen.

Die erfindungsgemäße Verbindungsvorrichtung hat demnach eine Kuppeleinrichtung, die während des Ein-/Ausführens eine im Vergleich zu herkömmlichen Kuppeleinrichtungen verminderte effektive Querschnittsfläche bezogen auf die Wegrichtung ihres dazu zurückzulegenden Weges aufweist. Die Begriffe Innenteil und Außenteil sind daher im Sinne der Erfindung nicht so zu verstehen, daß der Außenteil der Innenteil vollständig umschließen muß, sondern derart, daß es jedenfalls eine Ebene gibt, bezüglich der eine Projektion der Kuppeleinrichtung auf die Ebene bei festgelegter Orientierung der Kuppeleinrichtung gegenüber einer entsprechenden Projektion des Innenteils auf die Ebene bei gleicher Orientierung so ausgelegt ist, daß bezüglich mindestens einer in der Ebene liegenden und die Projektionen schneidenden Achse eine Abmessung der Projektion der Kuppeleinrichtung größer als eine entsprechende Abmessung der Projektion des Innenteils ist.

Da das zumindest abschnittsweise Anbringen (z. B. Einstecken) des Innenteils in den Außenteil von einer Seite erfolgt, die nicht mit der Koppelseite des Außenteils zusammenfällt, wird erreicht, daß der Außenteil nicht auf das Kabel aufgefädelt werden muß, um ihn an dem Innenteil anzubringen. Ansonsten müßte der Außenteil nach dem oben beschriebenen Einsatz doch durch die Öffnung(en) des Patientenkörpers durchgeführt werden.

Das Kabel stellt also für den Außenteil eine Art topologisches Hindernis dar. Für die Verbindungsvorrichtung ist daher bevorzugt vorgesehen, daß der Außenteil ausgehend von einem Trennzustand der Kuppeleinrichtung, in dem er das Kabel nicht bezüglich dessen radialer Richtung umschließt, zur Herstellung eines Verbindungszustands der Kuppeleinrichtung an dem Innenteil angebracht werden kann, ohne daß er dazu von einem kuppeleinrichtungsfernen Ende des Kabels oder unter Ausführung einer Bewegungsumkehr von einem kuppeleinrichtungsnahen Ende des Kabels durchdrungen werden muß.

Es ist damit möglich, den Außenteil an dem Innenteil anzubringen, ohne ihn auf das Kabel auffädeln zu müssen. Ein schonendes Durchführen der Verbindungsvorrichtung durch den, in den bzw. aus dem menschlichen Körper ist somit wie in der oben erläuterten Weise möglich.

Weiter ist der Innenteil länglich ausgebildet, so daß er im Vergleich zu seiner Länge eine geringe Querabmessung hat. So wird für das Durchführen durch den Körper eine stromlinienförmige Form erreicht.

In einer vorteilhaften Ausgestaltung liegt die Querabmessung im Bereich 1 bis 12 mm, vorzugsweise 2 bis 10 mm, insbesondere 4 bis 8 mm. So kann ein nochmals schonenderer Einsatz der Verbindungsvorrichtung erreicht werden und insbesondere wird eine kleinere und somit ansehnlichere Ausgangsöffnung ermöglicht

Weiter liegt das Verhältnis der Querabmessung zu der entsprechenden Abmessung der Kuppeleinrichtung im Bereich von 10% bis 85%, vorzugsweise 30% bis 80%, insbesondere 50% bis 75%. Dies ermöglicht einerseits einen schonenden Einsatz der Verbindungsvorrichtung, wobei andererseits eine hohe Funktionalität der Kuppeleinrichtung sichergestellt wird.

Zweckmäßig weist der Innenteil und/oder die Kuppeleinrichtung einen kreisförmigen Querschnitt auf. So werden Kanten oder Ecken, die ein vergleichsweise hohes Verletzungsrisiko darstellen, weitgehend vermieden.

In einer besonders bevorzugten Ausführungsform umhüllt der Außenteil den Innenteil in einem/dem Verbindungszustand der Kuppeleinrichtung zumindest abschnittsweise. Somit beinhaltet der Außenteil eine Schutzfunktion für den Innenteil.

In einer besonders bevorzugten Ausführungsform ist der Innenteil in den Außenteil einsteckbar, und insbesondere aus ihm herausziehbar. Dies ermöglicht eine sehr einfache und zeitsparende Handhabung der Verbindungsvorrichtung.

Zweckmäßig ist ein Rastmechanismus vorgesehen, der einen Übergang von einem/dem Verbindungszustand in einen/den Trennzustand der Kuppeleinrichtung entgegenwirkt. So kann ein unerwünschtes und unbeabsichtigtes Lösen von Innen- und Außenteil weitgehend verhindert werden.

Zweckmäßig ist eine Führungseinrichtung vorgesehen, die einen Übergang von einem/dem Trennzustand in einen/den Verbindungszustand der Kuppeleinrichtung erleichtert. So kann die Handhabung der Verbindungsvorrichtung vereinfacht werden.

In einer vorteilhaften Ausführungsform ist eine Positioniereinrichtung vorgesehen, die bei einem Übergang von einem/dem Trennzustand in einen/den Verbindungszustand der Kuppeleinrichtung eine relative Positionierung des Innenteils gegenüber dem Außenteil ermöglicht. So kann der Innenteil automatisch so positioniert werden, daß das anschließende Koppeln mit dem passenden Gegenstück ohne weitere Maßnahmen möglich ist.

Erfindungsgemäß hat der Außenteil keinen unmittelbaren Anschluß an eine für elektrischen und/oder optischen Kontakt mit dem Gegenstück ausgelegte Kontaktseite des Innenteils, und der Innenteil kann insbesondere den Kontakt allein herstellen. Damit ist der Außenteil für den Kontakt selbst nicht erforderlich, und die Funktionen Kontakt bzw. Schutz des Kontakts sind in einfacher Weise getrennt vorgesehen.

In einer zweckmäßigen Ausgestaltung weist die Kontaktseite des Innenteils mehrere elektrische und/oder optische Kontakte, insbesondere drei elektrische Kontakte auf. So können einerseits mehrere unabhängige oder unterschiedliche Signale übertragen werden, insbesondere ist die Verbindungsvorrichtung an übliche medizinische einpflanzbare Apparate angepaßt.

Erfindungsgemäß weist der Außenteil eine insbesondere als Teil eines push-pull-Mechanismus ausgebildete Steckeinrichtung zum mechanischen Koppeln an das passende Gegenstück auf. So kann betreffend das Koppeln an das passende Gegenstück eine hohe Bedienerfreundlichkeit gewährleistet werden.

Zweckmäßig ist der Innenteil unlösbar mit dem Kabel verbunden. So weist die Vierbindungsvorrichtung eine überschaubar kleine Anzahl von Einzelteilen auf.

Zweckmäßig ist an einem koppelseitigen Abschnitt des Innenteils ein Schutzelement für dessen Kontaktseite vorgesehen, und insbesondere ragt der Außenteil im Verbindungszustand weiter in Richtung der Koppelseite, als das koppelseitige Ende des Schutzelementes. So kann einerseits die Kontaktseite des Innenteils allein z.B. von einer Knickschütztülle geschützt sein, während ein weitergehender Schutz im Verbindungszustand durch den Außenteil sichergestellt wird.

In einer besonders bevorzugten Ausführungsform kann ein Lösen des Außenteils von dem Innenteil in Richtung zu der Koppelseite des Außenteils erfolgen. So wird eine einfache Handhabung gewährleistet und damit die Bedienerfreundlichkeit der Verbindungsvorrichtung erhöht.

Darüber hinaus ist vorteilhaft vorgesehen, daß eine zum Lösen/Abziehen nötige Kraft in dem Bereich von 0,1 - 20 N, vorzugsweise 0,8 - 8 N, insbesondere 2,0 - 4,0 N liegt. So wird einerseits eine zufriedenstellende Verbindungskraft gegen unbeabsichtigtes Lösen zwischen Innen- und Außenteil gewährleistet, während der Bediener die Verbindung dennoch mit einem für ihn angenehmen Kraftaufwand lösen kann.

Zweckmäßig ist die Verbindungsvorrichtung dazu ausgelegt, Signale aus dem Körperinneren nach außen und umgekehrt übertragen zu können. So wird eine nicht nur einseitige sondern zweiseitige Kommunikation mit einem eingepflanzten medizinischen Apparat möglich.

Die Erfindung betrifft auch ein Verfahren zum Legen einer elektrischen und/oder optischen Verbindung aus einem Innenbereich des menschlichen Körpers nach außen mit der Möglichkeit eines anschließenden elektrischen und/oder optischen Koppelns der Verbindung durch die Kuppeleinrichtung der erfindungsgemäßen Verbindungsvorrichtung mit einem passenden Gegenstück, bei dem das Innenteil der Kuppeleinrichtung von dem Körperinnenbereich nach außen bewegt wird, wobei nach der Bewegung und vor dem Koppeln mit dem passenden Gegenstück der Innenteil und ein mit dem Innenteil verbindbarer Außenteil der Kuppeleinrichtung zu dem Verbindungszustand der Kuppeleinrichtung zusammengesetzt wird.

Die bei diesem Verfahren gegenüber denjenigen des Stands der Technik auftretenden Vorteile sind bereits oben erläutert worden. Zusammengefaßt läßt sich sagen, daß gemäß der Erfindung nur der Innenteil der Kuppeleinrichtung aus dem Innenbereich des menschlichen Körpers nach außen bewegt wird, um einen schonenden Eingriff zu ermöglichen, wobei der Innenteil während des Legens der Verbindung weniger geschützt ist als bei herkömmlichen Verfahren. Dies spielt aber keine Rolle, weil der Schutz nur während des Legens der Verbindung und nicht während des Betriebs verringert ist.

Schließlich sieht die Erfindung gemäß den obigen Erläuterungen eine Verwendung einer Kuppeleinrichtung von der Erfindungsgemäßen Verbindungsvorrichtung mit den von ihrem Innenteil lösbaren Außenteil zum Einführen des Innenteils in das Innere eines Patientenkörpers durch eine erste Öffnung und zum Ausführen des Innenteils aus dem Inneren des Patientenkörpers durch eine zweite Öffnung vor, wobei die Kuppeleinrichtung zum Einführen und/oder Ausführen von einem/dem Verbindungszustand in einen/den Trennzustand gebracht werden kann.

Weitere Einzelheiten und Vorteile der Erfindung ergeben sich aus der folgenden Beschreibung mit Bezug auf die beigelegten Figuren, von denen
- Fig. 1: eine perspektivische Darstellung einer Ausführungsform der erfindungsgemäßen Verbindungsvorrichtung in einem Verbindungszustand ist;
- Fig. 2: einen Längsschnitt eines Innenteils der Kuppeleinrichtung der in Fig. 1 gezeigten Ver- bindungsvorrichtung (aber in einem Trennzustand) sowie eine Außenansicht deren Außenteils zeigt;
- Fig. 3: eine Stirnansicht eines koppelseitigen Endbereichs des in Fig. 2 gezeigten Innenteils ist; und
- Fig. 4: eine teilweise weggeschnittene Teilansicht der in Fig. 1 gezeigten Verbindungs- vorrichtung ist, die mit einem passenden Gegenstück gekoppelt ist, wobei ein Teil des Außenteils und ein Teil des Gegenstücks in einer Längsschnittansicht dargestellt sind.

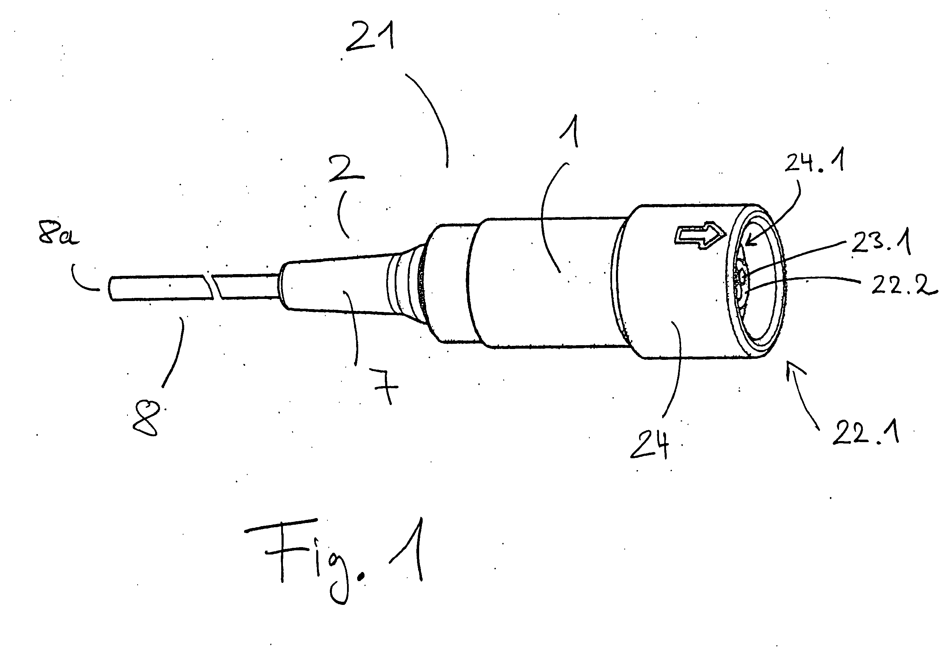

In Fig. 1 ist ein Ausführungsbeispiel für eine erfindungsgemäße Verbindungsvorrichtung dargestellt, wobei eine Kuppeleinrichtung 21 der Verbindungsvorrichtung in einem Verbindungszustand vorliegt. Man erkennt in Fig. 1 den Außenteil 1 der Kuppeleinrichtung 21, und ein auf einem kabelfernen Endbereich 24 des Außenteils 1 gezeichneter Pfeil zeigt in Richtung auf die Koppelseite 22.1 des Außenteils 1. Im Verbindungszustand ist ein Innenteil 2 der Kuppeleinrichtung 21 abschnittsweise im Inneren des Außenteils 1 angeordnet bzw. von dem Außenteil 1 umhüllt.

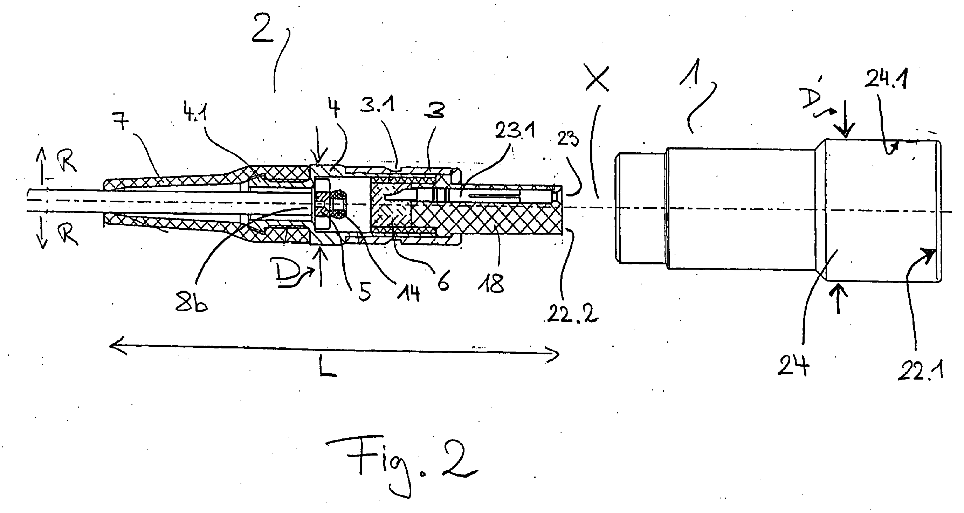

Betreffend den Innenteil 2 sind in Fig. 1 zur Koppelseite 22.1 des Außenteils 1 hin lediglich ein koppelseitiges Ende 22.2 des Innenteils 2 mit einem Ausgang eines elektrischen Kontaktes 23.1 der Kontaktseite 23 des Innenteils 2 sowie ein kabelseitiger Endbereich des Innenteils 2 sichtbar, der in diesem Ausführungsbeispiel als Knickschutztülle 7 ausgebildet ist, welche den Kabelanschlußbereich gegen Abknicken schützt. Weiter ist ein Kabel 8 zu sehen, das in diesem Ausführungsbeispiel elektrische Signale übertragen kann, wobei dessen kuppeleinrichtungsnahes Ende 8b (Fig. 2) in Fig. 1 von dem Innenteil 2 verdeckt ist, und dessen kuppeleinrichtungsfernes Ende 8a abgetrennt dargestellt ist. Letzteres deutet an, daß das Kabel 8 länger ist als in Fig. 1 gezeigt.

Wie gut aus Fig. 1 zu erkennen ist, sind sowohl die Kuppeleinrichtung 21 als auch der Innenteil 2 länglich gebaut, und beide weisen einen kreisförmigen Querschnitt auf, der allerdings längs der Längsrichtung unterschiedlich groß ausfallen kann. So ist etwa der koppelseitige Bereich 24 des Außenteils 1 mit dem größten Durchmesser bzw. der größten Querabmessung versehen, da innerhalb des Bereiches 24 Raum zur Ankopplung eines passenden Gegenstücks der Verbindungsvorrichtung freigehalten ist.

Die Kuppeleinrichtung 21 kann von dem in Fig. 1 gezeigten Verbindungszustand in den in Fig. 2 gezeigten Trennzustand gebracht werden, indem einfach der Außenteil 1 von dem Innenteil 2 in Richtung des Pfeils abgezogen wird, d.h. zur Koppelseite 22.1 des Außenteils hin. Umgekehrt kann in diesem Ausführungsbeispiel die Kuppeleinrichtung 21 aus dem in Fig. 2 dargestellten Trennzustand wieder in den in Fig. 1 dargestellten Verbindungszustand gebracht werden, indem der Innenteil 2 von der der Koppelseite 22.1 des Außenteils 1 entgegengesetzten Seite (wieder in Richtung des Pfeils) in den Außenteil 1 eingesteckt wird. Demnach wird der Innenteil 2 von einer nicht mit der Koppelseite 22.1 des Außenteils 1 zusammenfallenden Seite her an dem Außenteil 1 angebracht bzw. darin eingesteckt.

In Fig. 2 ist ein Trennzustand der Kuppeleinrichtung 21 dargestellt, in dem der Außenteil 1 das Kabel 8 nicht bezüglich dessen radialer Richtung R umschließt. Der Außenteil 1 kann entgegen der Richtung des Pfeils aus Fig. 1 an dem Innenteil 2 angebracht werden, ohne daß er vorher auf das Kabel 8 aufgefädelt werden muß. Das heißt, der Außenteil 1 muß weder von dem kuppeleinrichtungsfernen Ende 8a des Kabels 8 durchdrungen werden (Auffädelung von der Seite des entfernten Kabelendes), noch unter Ausführung einer Bewegungsumkehr von dem kuppeleinrichtungsnahen Ende 8b des Kabels 8 durchdrungen werden.

In Fig. 2 ist neben dem Außenteil 1 auch der Innenteil 2 in einer Längsschnittansicht dargestellt. Man erkennt, daß ein Hauptkörper des Innenteils 2 aus einer vorderen Hülse 3 und einer hinteren Hülse 4 aufgebaut ist. Die hintere Hülse 4 verjüngt sich zum kabelseitigen Ende, um schließlich an ihrem Endbereich einen ringförmigen Vorsprung 4.1 zu bilden. Der ringförmige Vorsprung 4.1 dient dazu, daß die Knickschutztülle 7 nach Montage des Innenteils 2 nicht mehr von diesem lösbar ist, so daß die Knickschutztülle nicht als weiteres Einzelteil einer Verlustgefahr ausgesetzt ist. Das Kabel 8 ist über sein kuppeleinrichtungsnahes Ende 8b über eine Anschlußeinrichtung innerhalb der hinteren Hülse 4 durch einen Ring 5 und eine Kugel 14 befestigt. Die drei Pole des Kabels 8 sind mit der Kontaktseite 23 des Innenteils 2 verbunden, wobei in Fig. 2 nur ein elektrischer Kontakt 23.1 der Kontaktseite 23 sichtbar ist.

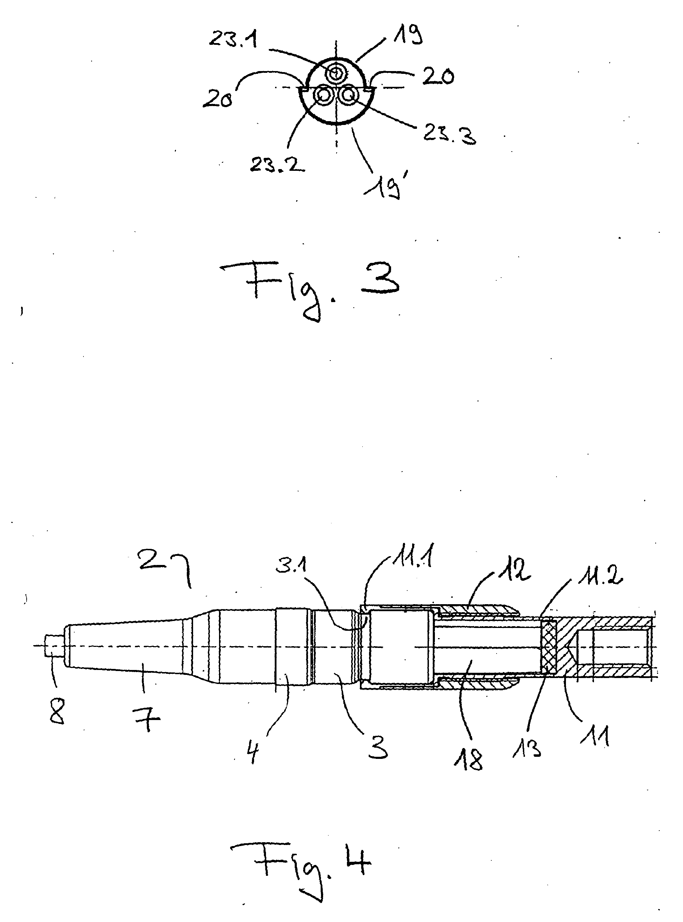

Die vordere Hülse 3 hat an ihrem kabelfernen Ende eine Öffnung, aus der sich ein Schutzelement 18 in Richtung der Koppelseite 22 erstreckt, welches die drei elektrischen Kontakte 23.1, 23.2 und 23.3 schützend umgibt, wie auch in Fig. 3 in einer Stirnansicht zu sehen ist. Der Innenraum des Innenteils 2 zwischen der Anschlußeinrichtung und dem Schutzelement 18 weist eine Vergußhülse 6 auf. Man kann erkennen, daß der Außenteil 1 nicht in das elektrische Kontaktieren zwischen Innenteil 2 und Gegenstück involviert ist.

Die Knickschutztülle 7 ist vorzugsweise aus einem harten Gummimaterial hergestellt, die vordere Hülse 3 und hintere Hülse 4 vorzugsweise aus einem festen Stoff wie etwa einem Metall hergestellt, und das Schutzelement 18 ist vorzugsweise aus einem festen, aber bei der Herstellung leicht formbaren Material wie Hartplastik hergestellt.

Wie gut in Fig. 2 zu sehen ist, kann der Innenteil 2 zu einem Teil seiner Länge L entlang einer zentralen Achse X in den Außenteil 1 eingesteckt werden. Ebenfalls ist gut zu erkennen, daß der Durchmesser bzw. die Querabmessung D des Innenteils 2 deutlich kleiner ist als die entsprechende (größte) Querabmessung D' des Außenteils 1, die zugleich die größte Querabmessung der Kuppeleinrichtung 21 darstellt. In dem gezeigten Ausführungsbeispiel beträgt der Durchmesser D des Innenteils 2 etwa 7 mm, und das Verhältnis der Durchmesser bzw. Querabmessungen D/D' beträgt ca. 57%. Die Kuppeleinrichtung 21 ist daher so ausgelegt, daß der Innenteil 2 eine auf die Achse X bezogene Querschnittsfläche aufweist, die lediglich noch 1/3 der entsprechenden Querschnittsfläche der Kuppeleinrichtung 21 im Trennzustand beträgt. Die erfindungsgemäße Verbindungsvorrichtung kann daher erheblich schonender eingesetzt werden als herkömmliche Verbindungsvorrichtungen mit Kuppeleinrichtungen vergleichbarer Abmessungen.

Eine ringförmige Einkerbung 3.1 der vorderen Hülse 3 des Innenteils 2 ist ein Teil eines Rastmechanismus, der den Innenteil 2 in dem Außenteil 1 einrasten läßt. Dies ist besser in Fig. 4 zu sehen, wo neben dem Innenteil 2 noch ein Bereich des Außenteils 1 abgebildet ist, der in den Figuren 1 und 2 nicht zu sehen ist. Dabei handelt es sich um eine Überwurfmutter 12, die innerhalb eines mittleren Abschnitts des Außenteils 1 angebracht ist. Die Überwurfmutter 12 ist so ausgelegt, daß sie einen nach innen vorragenden Ringflansch 11.1 in die ringförmige Einkerbung 3.1 drängt. Der Ringflansch 11.1 bildet damit zusammen mit der ringförmigen Einkerbung 3.1 einen Rastmechanismus, der die Kuppeleinrichtung 21 vor einem unbeabsichtigten Übergang in den Trennzustand schützt. In dem konkreten Ausführungsbeispiel ist zum Lösen des Rastmechanismus eine Kraft von ca. 3 N erforderlich.

Ebenfalls in Fig. 4 dargestellt ist ein Teil eines passenden Gegenstücks der Verbindungsvorrichtung, der in Form einer Klauenhülse 11 gebildet ist. Dabei weist die Klauenhülse 11 eine dem koppelseitigen Ende 22.2 des Schutzelementes 18 des Innenteils 2 zugewandte Flachdichtung 13 auf. Nicht in Fig. 4 dargestellt sind drei elektrische Kontaktstifte, die sich aus der Klauenhülse 11 in Richtung des Innenteils 2 der Kuppeleinrichtung 21 erstrecken, und zum Einführen in Aufnahmebereiche der elektrischen Kontakte 23.1, 23.2, 23.3 der Kontaktseite 23 des Innenteils ausgelegt sind.

In Fig. 3, die eine Stirnansicht des Schutzelements 18 des Innenteils 2 zeigt, wobei die elektrischen Kontakte 23 als Kreisringe erscheinen, die Aufnahmebereiche zur Aufnahme der elektrischen Kontaktstifte des Gegenstücks definieren, ist ebenfalls zu sehen, daß das Schutzelement 18 einen im wesentlichen kreisförmigen Querschnitt hat. Genauer gesagt setzt sich der Querschnitt des Schutzelements 18 aus zwei Halbkreisflächen mit unterschiedlichen Radien zusammen, so daß das Schutzelement 18 unterschiedlich ausgebildete Zylinderflächenteilbereiche 19, 19' aufweist, die längs der zentralen Achse X, die in Fig. 3 senkrecht auf der Papierebene steht, über Kantenbereiche 20 miteinander verbunden sind. Der Öffnungsbereich der vorderen Hülse 3, aus dem sich das Schutzelement 18 erstreckt, weist dieselbe Form und Größe auf wie der in Fig. 3 abgebildete Querschnitt des Schutzelements 18. Ebenfalls weist ein entfernter Endbereich der Überwurfmutter 12 (auf der dem Ringflansch 11.1 entgegengesetzten Seite) einen nach innen ragenden leicht nachgiebigen Flanschbereich auf, der ebenfalls eine Öffnung von genau der Form freilässt, die dem in Fig. 3 abgebildeten Querschnitt entspricht. Dies ist allerdings in Fig. 4 nicht dargestellt.

Die oben erläuterte übereinstimmende Form des Querschnitts des Schutzelements 18 stellt eine Führungseinrichtung dar, mittels der sich das Schutzelement 18 des Innenteils 2 und damit der Innenteil 2 einfach in den Außenteil 1 der Kuppeleinrichtung 21 einstecken läßt. Darüber hinaus sorgen die Längskanten 20 zwischen den Bereichen 19 des Schutzelements 18 für eine Positioniereinrichtung, die die genaue Lage der elektrischen Kontakte 23 bezüglich des Außenteils 1 der Kuppeleinrichtung 21 festlegt. Bei entsprechender Auslegung eines Klauenbereichs 11.2 der Klauenhülse 11 des Gegenstücks sind die Kontaktstifte des Gegenstücks automatisch richtig positioniert, um bei einem Ankoppeln der Klauenhülse 11 an die Kontaktseite 23 des Innenteils 2 automatisch richtig in die Aufnahmebereiche der elektrischen Kontakte 23.1, 23.2 und 23.3 eingesteckt werden zu können.

Aus Fig. 1 geht weiter andeutungsweise hervor, daß der Außenteil 1 auf der Innenseite seines koppelseitigen Bereichs 24 (dem Bereich mit der größten Querabmessung D') eine ringförmige Einkerbung 24.1 aufweist. Diese ringförmige Einkerbung 24.1 stellt eine Steckeinrichtung dar, die dem mechanischen Koppeln an das passende Gegenstück dient. Sie kann beispielsweise als Teil eines push-pull-Mechanismus ausgebildet sein, und erlaubt demnach die gleiche mechanische Koppelqualität der Kuppeleinrichtung 21, die auch herkömmliche Kuppeleinrichtungen aufweisen, d.h., die gemäß der Erfindung vorgesehene Möglichkeit der Abtrennung des Außenteils 1 vom Innenteil 2 der Kuppeleinrichtung 21 beeinträchtigt nicht deren Qualität zur Kopplung an das passende Gegenstück.

Das in den Fig. 1 bis 4 dargestellte Ausführungsbeispiel der vorliegenden Erfindung ist lediglich als Beispiel für die in den Ansprüchen geschützte Erfindung und daher nicht als schutzbeschränkend anzusehen. Vielmehr können die in der obigen Beschreibung sowie in den Ansprüchen offenbarten Merkmale der Erfindung sowohl einzeln als auch in beliebiger Kombination für die Verwirklichung der Erfindung in ihren verschiedenen Ausführungsformen wesentlich sein.

## Patentansprüche

1. Verbindungsvorrichtung für medizinische Zwecke zur Übertragung optischer und/oder elektrischer Signale zwischen einem Körperinneren und einem Körperäußeren mit
einem Kabel (8) und
einer Kuppeleinrichtung (21), die zum Koppeln mit einem passenden Gegenstück (11) an einer Koppelseite (22) ausgelegt ist, wobei die Koppeleinrichtung (21)
einen Innenteil (2) mit einer Anschließeinrichtung (5, 14) zum Anschließen an das Kabel (8) und
einen Außenteil (1) aufweist, der eine Steckeinrichtung (24.1) zum mechanischen Koppeln an das passende Gegenstück aufweist und keinen unmittelbaren Anschluß an eine für e-lekrischen und/oder optischen Kontakt mit dem Gegenstück (11) ausgelegte Kontaktseite (23) des Innenteils (2) hat,
wobei der Außenteil (1) lösbar an dem Innenteil (2) angebracht ist,
der Innenteil (2) von einer nicht mit der Koppelseite (22.1) des Außenteils (1) zusammenfallenden Seite her an dem Außenteil (1) anbringbar ist, und
der Innenteil (2) länglich mit einer im Vergleich zur'Länge (L) geringeren Querabmessung (D) ausgebildet ist,
wobei das Verhältnis der Querabmessung (D) zu der entsprechenden Abmessung (D') der Kuppeleinrichtung im Bereich von 10 % bis 85 % liegt.

2. Verbindungsvorrichtung nach Anspruch 1,
**dadurch gekennzeichnet, daß**
der Außenteil (1) ausgehend von einem Trennzustand der Kuppeleinrichtung (21), in dem er das Kabel (8) nicht bezüglich dessen radialer Richtung (R) umschließt, zur Herstellung eines Verbindungszustands der Kuppeleinrichtung (21) an dem Innenteil (2) angebracht werden kann, ohne daß er dazu von einem kuppeleinrichtungsfernen Ende (8a) des Kabels (8) oder unter Ausführung einer Bewegungsumkehr von einem kuppeleinrichtungsnahen Ende (8b) des Kabels (8) durchdrungen werden muß,

3. Verbindungsvorrichtung nach Anspruch 1 oder 2, **dadurch gekennzeichnet, daß** die Querabmessung (D) im Bereich 1 bis 12 mm, vorzugsweise 2 bis 10 mm, insbesondere 4 bis 8 mm liegt.

4. Verbindungsvorrichtung nach einem der vorhergehenden Ansprüche, **dadurch gekennzeichnet, daß** das Verhältnis der Querabmessung (D) zu der entsprechenden Abmessung (D') der Kuppeleinrichtung im Bereich von 30% bis 80%, insbesondere 50% bis 75% liegt.

5. Verbindungsvorrichtung nach einem der vorhergehenden Ansprüche, **dadurch gekennzeichnet, daß** der Innenteil (2) und/oder die Kuppeleinrichtung (21) einen kreisförmigen Querschnitt aufweist.

6. Verbindungsvorrichtung nach einem der vorhergehenden Ansprüche, **dadurch gekennzeichnet, daß** der Außenteil (1) den Innenteil (2) in einem/dem Verbindungszustand der Kuppeleinrichtung (21) zumindest abschnittsweise umhüllt.

7. Verbindungsvorrichtung nach einem der vorhergehenden Ansprüche, **dadurch gekennzeichnet, daß** der Innenteil (2) in den Außenteil (1) einsteckbar ist und insbesondere aus ihm herausziehbar ist.

8. Verbindungsvorrichtung nach einem der vorhergehenden Ansprüche, **gekennzeichnet durch** einen Rastmechanismus (3.1, 12.1), der einem Übergang von einem/dem Verbindungszustand in einen/den Trennzustand der Kuppeleinrichtung (21) entgegenwirkt.

9. Verbindungsvorrichtung nach einem der vorhergehenden Ansprüche, **gekennzeichnet durch** eine Führungseinrichtung (19, 19', 20), die einen Übergang von einem/dem Trennzustand in einen/den Verbindungszustand der Kuppeleinrichtung (21) erleichtert.

10. Verbindungsvorrichtung nach einem der vorhergehenden Ansprüche, **gekennzeichnet durch** eine Positioniereinrichtung (20), die bei einem Übergang von einem/dem Trennzustand in einen/den Verbindungszustand der Kuppeleinrichtung (21) eine relative Positionierung des Innenteils (2) gegenüber dem Außenteil (1) ermöglicht.

11. Verbindungsvorrichtung nach einem der vorhergehenden Ansprüche, **dadurch gekennzeichnet, daß** der Innenteil (2) allein den elektrischen und/oder optischen Kontakt mit dem Gegenstück (11) herstellen kann.

12. Verbindungsvorrichtung nach einem der vorhergehenden Ansprüche, **dadurch gekennzeichnet, daß** eine/die Kontaktseite (23) des Innenteils (2) mehrere elektrische und/oder optische Kontakte, insbesondere drei elektrische Kontakte (23.1, 23.2, 23.3) aufweist.

13. Verbindungsvorrichtung nach einem der vorhergehenden Ansprüche, **dadurch gekennzeichnet, daß** die Steckeinrichtung (24.1) als Teil eines push-pull-Mechanismus ausgebildet ist.

14. Verbindungsvorrichtung nach einem der vorhergehenden Ansprüche, **dadurch gekennzeichnet, daß** der Innenteil (2) unlösbar mit dem Kabel (8) verbunden ist.

15. Verbindungsvorrichtung nach einem der vorhergehenden Ansprüche, **dadurch gekennzeichnet, daß** an einem koppelseitigen Abschnitt des Innenteils (2) ein Schutzelement (18) für dessen Kontaktseite (23) vorgesehen ist, und insbesondere der Außenteil (1) im Verbindungszustand weiter in Richtung der Koppelseite (22.1) ragt als das koppelseitige Ende (22.2) des Schutzelementes.

16. Verbindungsvorrichtung nach einem der vorhergehenden Ansprüche, **dadurch gekennzeichnet, daß** ein Lösen des Außenteils (1) von dem Innenteil (2) in Richtung zu der Koppelseite (22.1) des Außenteils (1) erfolgen kann.

17. Verbindungsvorrichtung nach Anspruch 16, **dadurch gekennzeichnet, daß** eine zum Lösen/Abziehen nötige Kraft in dem Bereich von 0,1 bis 20 N, vorzugsweise 0,8 - 8 N, insbesondere 2,0 - 4,0 N liegt.

18. Verbindungsvorrichtung nach einem der vorhergehenden Ansprüche, **dadurch gekennzeichnet, daß** sie dazu ausgelegt ist, die Signale aus dem Körperinneren nach außen und umgekehrt zu übertragen.

19. Verfahren zum Legen einer elektrischen und/oder optischen Verbindung aus einem Innenbereich des menschlichen Körpers nach außen mit der Möglichkeit eines anschließenden elektrischen und/oder optischen Koppelns der Verbindung durch eine Kuppeleinrichtung (21) einer Verbindungsvorrichtung nach einem der vorhergehenden Ansprüche mit einem passenden Gegenstück (11), bei dem der Innenteil (2) der Kuppeleinrichtung (21) von dem Körperinnenbereich nach außen bewegt wird und bei dem nach dieser Bewegung und vor dem Koppeln mit dem passenden Gegenstück (11) der Innenteil (2) und der mit dem Innenteil verbindbare Außenteil (1) der Kuppeleinrichtung (21) in den Verbindungszustand der Kuppeleinrichtung (21) zusammengesetzt werden.

20. Verwendung einer Kuppeleinrichtung (21) einer Verbindungsvorrichtung nach einem der Ansprüche 1 bis 18 zum Einführen des Innenteils (2) in das Innere eines Patientenkörpers durch eine erste Öffnung und zum Ausführen des Innenteils (2) aus dem Inneren des Patientenkörpers durch eine zweite Öffnung, wobei die Kuppeleinrichtung (21) zum Einführen und/oder Ausführen von einem/dem Verbindungszustand durch Lösen des Außenteils (1) in einen/den Trennzustand gebracht werden kann.

## Claims

1. A connecting device for medical purposes for transferring optical and/or electrical signals between the inside and the outside of a body, comprising
a cable (8) and
a coupling device (21) designed for coupling to a matching counterpart (11) on a coupling side (22), the coupling device (21)
having an inner part (2) with an attaching device (5, 14) for attaching to the cable (8), and
an outer part (1) which has a plugging device (24.1) for mechanically coupling to the matching counterpart and no direct attachment to a contact side (23) of the inner part (2) for electrical and/or optical contact with the counterpart (11),
the outer part (1) being fitted detachably to the inner part (2),
the inner part (2) being fittable to the outer part (1) from a side not coinciding with the coupling side (22.1) of the outer part (1), and
the inner part (2) being formed longitudinally with a smaller lateral dimension (D) in comparison with the length (L),
the ratio of the lateral dimension (D) to the corresponding dimension (D') of the coupling device coming within the range of 10% to 85%.

2. The connecting device according to Claim 1,
**characterised in that**
starting from a separating state of the coupling device (21) in which it does not surround the cable (8) with regard to the radial direction (R) of the latter, the outer part (1) can be fitted to the inner part (2) in order to establish a connecting state of the coupling device (21), without it for this purpose having to be penetrated by an end (8a) of the cable (8) spaced apart from the coupling device or by an end (8b) of the cable (8) close to the coupling device thus implementing a movement reversal.

3. The connecting device according to Claim 1 or 2, **characterised in that** the lateral dimension (D) comes within the range of 1 to 12 mm, preferably 2 to 10 mm, in particular 4 to 8 mm.

4. The connecting device according to any of the preceding claims, **characterised in that** the ratio of the lateral dimension (D) to the corresponding dimension (D') of the coupling device comes within the range of 30% to 80%, in particular 50% to 75%.

5. The connecting device according to any of the preceding claims, **characterised in that** the inner part (2) and/or the coupling device (21) has a circular cross-section.

6. The connecting device according to any of the preceding claims, **characterised in that** the outer part (1) envelops the inner part (2), at least in sections, in a/the connecting state of the coupling device (21).

7. The connecting device according to any of the preceding claims, **characterised in that** the inner part (2) can be plugged into the outer part (1) and in particular can be pulled out of the latter.

8. The connecting device according to any of the preceding claims, **characterised by** a locking mechanism (3.1, 12.1) which counters a crossover from a/the connecting state into a/the separating state of the coupling device (21).

9. The connecting device according to any of the preceding claims, **characterised by** a guide device (19, 19', 20) which facilitates a crossover from a/the separating state into a/the connecting state of the coupling device (21).

10. The connecting device according to any of the preceding claims, **characterised by** a positioning device (20) which upon a crossover from a/the separating state into a/the connecting state of the coupling device (21) enables relative positioning of the inner part (2) in relation to the outer part (1).

11. The connecting device according to any of the preceding claims, **characterised in that** the inner part (2) alone can establish the electrical and/or optical contact with the counterpart (11).

12. The connecting device according to any of the preceding claims, **characterised in that** a/the contact side (23) of the inner part (2) has a number of electrical and/or optical contacts, in particular three electrical contacts (23.1, 23.2, 23.3).

13. The connecting device according to any of the preceding claims, **characterised in that** the plugging device (24.1) is formed as part of a push/pull mechanism.

14. The connecting device according to any of the preceding claims, **characterised in that** the inner part (2) is connected undetachably to the cable (8).

15. The connecting device according to any of the preceding claims, **characterised in that** a protection element (18) for the contact side (23) of the inner part (2) is provided on a section on the coupling side of the latter, and in particular, in the connecting state the outer part (1) projects further towards the coupling side (22.1) than the end (22.2) on the coupling side of the protection element.

16. The connecting device according to any of the preceding claims, **characterised in that** the outer part (1) can be detached from the inner part (2) in the direction of the coupling side (22.1) of the outer part (1).

17. The connecting device according to Claim 16, **characterised in that** a force required to detach/pull off comes within the range of 0.1 to 20 N, preferably 0.8 - 8 N, in particular 2.0 - 4.0 N.

18. The connecting device according to any of the preceding claims, **characterised in that** it is designed to transfer the signals from the inside of the body to the outside and vice versa.

19. A method for placing an electrical and/or optical connection from an inner region of the human body to the outside with the possibility of subsequent electrical and/or optical coupling of the connection by means of a coupling device (21) of a connecting device according to any of the preceding claims to a matching counterpart (11), wherein the inner part (2) of the coupling device (21) is moved from the inner region of the body to the outside, and wherein after this movement and before coupling to the matching counterpart (11) the inner part (2) and the outer part (1) of the coupling device (21) connectable to the inner part are assembled to form the connecting state of the coupling device (21).

20. The use of a coupling device (21) of a connecting device according to any of Claims 1 to 18 for introducing the inner part (2) into the inside of a patient's body via a first opening and for moving the inner part (2) out of the inside of the patient's body via a second opening, it being possible to bring the coupling device (21) for moving into and/or moving out from a/the connecting state by detaching the outer part (1) into a/the separating state.

## Revendications

1. Dispositif de raccord utilisé à des fins médicales pour transmettre des signaux optiques et/ou électriques entre l'intérieur d'un corps et l'extérieur d'un corps, comportant
un câble (8) et
un dispositif de couplage (21) conçu pour se coupler à une pièce complémentaire (11) adaptée sur un côté de couplage (22), le dispositif de couplage (21) présentant une partie intérieure (2) comportant un dispositif de connexion (5, 14) destiné à se connecter au câble (8) et
une partie extérieure (1) présentant un dispositif d' enfichage (24.1) conçu pour se coupler mécaniquement à la pièce complémentaire adaptée et ne possédant pas de connexion directe à un côté de contact (23) de la partie intérieure (2) conçu pour un contact électrique et/ou optique avec la pièce complémentaire (11),
la partie extérieure (1) étant agencée de manière amovible sur la partie intérieure (2),
la partie intérieure (2) étant mise en place sur la partie extérieure (1) depuis un côté ne coïncidant pas avec le côté de couplage (22.1) de la partie extérieure (1), et
la partie intérieure (2) étant conçue de manière allongée, en présentant une dimension transversale (D) relativement plus petite que la longueur (L),
le rapport entre la dimension transversale (D) et la dimension correspondante (D') du dispositif de couplage se situant dans la plage de 10 % à 85 %.

2. Dispositif de raccord selon la revendication 1,
**caractérisé en ce que**
la partie extérieure (1) peut être appliquée à la partie intérieure (2), en partant d'un état de déconnexion du dispositif de couplage (21) dans lequel elle n'entoure pas le câble (8) relativement à sa direction radiale (R), pour produire un état de connexion du dispositif de couplage (21) sans pour cela devoir être traversée par une extrémité distale (8a) du câble (8) par rapport au dispositif de couplage ou, lorsque le mouvement est inversé, par une extrémité proximale (8b) du câble (8) par rapport au dispositif de couplage.

3. Dispositif de raccord selon la revendication 1 ou 2, **caractérisé en ce que** la dimension transversale (D) se situe dans la plage de 1 à 12 mm, de préférence de 2 à 10 mm, et notamment de 4 à 8 mm.

4. Dispositif de raccord selon l'une des revendications précédentes, **caractérisé en ce que** le rapport entre la dimension transversale (D) et la dimension correspondante (D') du dispositif de couplage se situe dans la plage de 30 % à 80 %, notamment de 50 % à 75 %.

5. Dispositif de raccord selon l'une des revendications précédentes, **caractérisé en ce que** la partie intérieure (2) et/ou le dispositif de couplage (21) présentent une section transversale circulaire.

6. Dispositif de raccord selon l'une des revendications précédentes, **caractérisé en ce que** la partie extérieure (1) enveloppe au moins partiellement la partie intérieure (2) dans un/dans l'état de connexion du dispositif de couplage (21).

7. Dispositif de raccord selon l'une des revendications précédentes, **caractérisé en ce que** la partie intérieure (2) est susceptible d'être enfichée dans la partie extérieure (1) et est notamment susceptible d'en être retirée.

8. Dispositif de raccord selon l'une des revendications précédentes, **caractérisé par** un mécanisme d'encliquetage (3.1,12.1) s'opposant au passage d'un/de l'état de connexion à un/à l'état de déconnexion du dispositif de couplage (21).

9. Dispositif de raccord selon l'une des revendications précédentes, **caractérisé par** un dispositif de guidage (19, 19', 20) facilitant le passage d'un/de l'état de déconnexion à un/à l'état de connexion du dispositif de couplage (21).

10. Dispositif de raccord selon l'une des revendications précédentes, **caractérisé par** un dispositif de positionnement (20) permettant un positionnement relatif de la partie intérieure (2) par rapport à la partie extérieure (1) lors d'un passage d'un/de l'état de déconnexion à un/à l'état de connexion du dispositif de couplage (21).

11. Dispositif de raccord selon l'une des revendications précédentes, **caractérisé en ce que** la partie intérieure (2) est en mesure de réaliser seule le contact électrique et/ou optique avec la pièce complémentaire (11).

12. Dispositif de raccord selon l'une des revendications précédentes, **caractérisé en ce qu'**un/le côté de contact (23) de la partie intérieure (2) présente plusieurs contacts électriques et/ou optiques, notamment trois contacts électriques (23.1, 23.2, 23.3).

13. Dispositif de raccord selon l'une des revendications précédentes, **caractérisé en ce que** le dispositif d'enfichage (24.1) est conçu comme partie intégrante d'un mécanisme push-pull.

14. Dispositif de raccord selon l'une des revendications précédentes, **caractérisé en ce que** la partie intérieure (2) est raccordée au câble (8) de manière inamovible.

15. Dispositif de raccord selon l'une des revendications précédentes, **caractérisé en ce qu'**il est prévu, au niveau d'une section située du côté de couplage de la partie intérieure (2), un élément de protection (18) pour son côté de contact (23), la partie extérieure (1) faisant notamment davantage saillie, à l'état de connexion, dans la direction du côté de couplage (22.1) que ne le fait l'extrémité côté de couplage (22.2) de l'élément de protection.

16. Dispositif de raccord selon l'une des revendications précédentes, **caractérisé en ce qu'**un détachement de la partie extérieure (1) par rapport à la partie intérieure (2) peut s'effectuer dans la direction du côté de couplage (22.1) de la partie extérieure (1).

17. Dispositif de raccord selon la revendication 16, **caractérisé en ce qu'**une force nécessaire au détachement/retrait se situe dans la plage de 0,1 à 20 N, de préférence de 0,8 à 8 N, notamment de 2,0 à 4,0 N.

18. Dispositif de raccord selon l'une des revendications précédentes, **caractérisé en ce qu'**il est conçu pour transmettre les signaux de l'intérieur vers l'extérieur du corps et vice-versa.

19. Procédé de mise en place d'un raccord électrique et/ou optique partant de l'intérieur du corps humain vers l'extérieur offrant la possibilité d'un couplage électrique et/ou optique consécutif du raccord, au moyen d'un dispositif de couplage (21) appartenant à un dispositif de raccord selon l'une des revendications précédentes, avec une pièce complémentaire (11) adaptée, dans lequel une partie intérieure (2) du dispositif de couplage (21) est déplacée depuis l'intérieur du corps vers l'extérieur et dans lequel, à la suite de ce déplacement et préalablement au couplage avec la pièce complémentaire (11) adaptée, la partie intérieure (2) et une partie extérieure (1) du dispositif de couplage (21) qui est destinée à être raccordée à la partie intérieure sont assemblées pour former un/l'état de connexion du dispositif de couplage (21).

20. Utilisation d'un dispositif de couplage (21) appartenant à un dispositif de raccord selon l'une des revendications 1 à 18 visant à introduire la partie intérieure (2) à l'intérieur du corps d'un patient par un premier orifice et à faire sortir la partie intérieure (2) de l'intérieur du corps du patient par un deuxième orifice, le dispositif de couplage (21) destiné à être introduit et/ou extrait pouvant passer d'un/de l'état de connexion à un/à l'état de déconnexion en détachant la partie extérieure (1).
